# EUROPÄISCHE PATENTSCHRIFT

(11) **EP 2 016 406 B1**
(45) Veröffentlichungstag und Bekanntmachung des Hinweises auf die Patenterteilung: **06.01.2010**
(21) Anmeldenummer: 07728720.9
(22) Anmeldetag: 02.05.2007
(51) Int. Cl.: G01N 33/487

(54) **DIAGNOSTISCHE TESTEINHEIT**
DIAGNOSTIC TEST UNIT
UNITE DE TEST DE DIAGNOSTIC

(30) Priorität: 06.05.2006 EP 06009396
(43) Veröffentlichungstag der Anmeldung: 21.01.2009
(73) Patentinhaber: F. Hoffmann-La Roche AG, 4070 Basel (CH); Roche Diagnostics GmbH, 68305 Mannheim (DE)
(72) Erfinder: SACHERER, Klaus-Dieter, 67281 Kirchheim (DE); DITSCHER, Wolfgang, 67691 Hochspeyer (DE); STEINBRÜCK, Ralf, 68305 Mannheim (DE); RUHL, Werner, 67117 Limburgerhof (DE); SCHÖTTLE, Klaus, 77731 Willstätt (DE)
(74) Vertreter: Pfiz, Thomas
(86) Internationale Anmeldenummer: PCT/EP2007/054267
(87) Internationale Veröffentlichungsnummer: WO 2007/128758

(56) Entgegenhaltungen:
- WO-A-2006/002432
- WO-A2-2006/059241
- US-A- 4 218 421

## Beschreibung

Die Erfindung betrifft eine diagnostische Testeinheit zur Untersuchung einer Körperflüssigkeit, insbesondere für Blutzuckertests nach dem Oberbegriff der Ansprüche 1 bzw. 6.

Solche Testeinheiten lassen sich vor allem zur Blutzuckerbestimmung als Verbrauchsmaterial in automatisch arbeitenden Handgeräten einsetzen, mit denen auch von Laien die erforderlichen Untersuchungsschritte einfach und schnell vorgenommen werden können. Auf dem aufgewickelten Analyseband sind eine Vielzahl von mit einer geeigneten Testchemie versehenen Testfeldern fortlaufend angeordnet. Alternativ lassen sich auch einzelne Teststreifen aus einem Magazin entnehmen. Die Körperflüssigkeit wird auf einem so bereitgestellten Testfeld appliziert, um sodann einen Nachweis vor Ort beispielsweise durch eine optische Untersuchung zu ermöglichen.

In der WO 2004/056269 A1 der Anmelderin sind verschiedene Dichtungskonzepte beschrieben, um den unverbrauchten Teil eines Testbands in einem Behältnis gegen schädliche Umwelteinflüsse zu schützen und zugleich einen Bandtransport für die sukzessive Bereitstellung der Testfelder zu erlauben. Hierbei wird das Testband über einen Durchlass zwischen Dichtung und einer Gehäusewand ausgegeben, wobei die Dichtelemente der profilierten Testfeldseite zugeordnet sind, während die Rückwand starr ist. Für die Montage bzw. Bestückung ist eine gesonderte Befüllmöglichkeit vorgesehen.

Für den Einsatz von Teststreifen beschreibt die EP-A-1321769 einen Spendeapparat, bei dem ein Einzelstreifen durch eine translatorische Bewegung zwischen einer Dichtung und einem starren Gehäusedeckel hindurch aus einem magazinierten Stapel heraus bereitgestellt wird. Zum Einfüllen eines Streifenstapels ist ein Schiebefach vorgesehen, das gesondert abgedichtet werden muss.

Aus der WO 2006/002432 A1 ist eine disposible Kassetteneinheit bekannt, welche ein Gehäuse, darin befindliche streifenförmige Testsensoren einen Ausgabemechanismus und eine bewegliche Dichtung umfasst. Die so genannte "Duckbill-Dichtung" bildet eine vorspringende umlaufende Dichtlippe, die sich unter leichter Verformung beidseitig an den ausgespendeten Teststreifen anlegt. Problematisch ist dabei die mangelhafte Anpresskraft, die mit zunehmender Gebrauchsdauer aufgrund Materialermüdung noch weiter nachlässt. Insbesondere bei unterschiedlichen Höhenprofilen des Testmaterials ist eine hinreichende Dichtfunktion nicht gewährleistet. Ein ähnliches Dichtkonzept mit lose gegeneinander anliegenden Dichtlippen ist auch bei einem Band mit elektrochemischen Sensoren aus der WO 2006/059241 A2 bekannt.

Ausgehend hiervon liegt der Erfindung die Aufgabe zugrunde, die im Stand der Technik aufgetretenen Nachteile zu vermeiden und eine gattungsgemäße Testeinheit für den disposiblen Einsatz in Handgeräten dahingehend zu verbessern, dass bei einfacher Herstellbarkeit eine hohe Lager- und Gebrauchsstabilität erreicht wird.

Zur Lösung dieser Aufgabe wird die im Patentanspruch 1 bzw. 6 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine Siegelfolie zugleich als Dichtelement zu nutzen. Dementsprechend wird gemäß der Erfindung vorgeschlagen, dass die Öffnung durch eine Verschlussfolie gegenüber der Umgebung des Behälters abgeschirmt ist, und dass das Testband bzw. der auszugebende Teststreifen durch einen Durchlassspalt zwischen der Verschlussfolie und der Dichtung hindurchgeführt ist. Auf diese Weise wird mit einfachen Mitteln eine Durchzugsdichtung geschaffen, wobei die Ausbildung eines Durchlassspalts zwischen flexibler Folie und vorzugsweise elastomerer Dichtung besondere Vorteile bei der Herstellung und im Gebrauch bietet. Durch das Aufsiegeln der Folie wird eine zuverlässige Feuchteabschirmung erreicht, wobei durch die Flexibilität auch höhenprofilierte Testmaterialien weitgehend ungehindert durchtreten können. Ein besonderer Vorteil ergibt sich dadurch, dass die versiegelbare Öffnung sowohl das Einfüllen als auch das Ausgeben der Test- und Hilfsmaterialien erlaubt, ohne dass zusätzliche Dichtmaßnahmen getroffen werden müssten. Gleichsam liegen also die Einfüll- und Ausgabeöffnung auf einer Ebene, die durch die Verschlussfolie dicht abgeschirmt wird.

Vorteilhafterweise ist das Testband vorzugsweise in Form eines Bandwickels durch die Öffnung hindurch in den Behälter eingesetzt, so dass sich die Bestückung und Abdichtung besonders einfach gestaltet.

Eine weitere Verbesserung hinsichtlich der Dichtwirkung lässt sich dadurch erreichen, dass das Testband zwischen aufeinander folgenden Testfeldern freie Trägerabschnitte aufweist, und dass im Lagerzustand ein testfeldfreier Trägerabschnitt in dem Durchlassspalt liegt.

In besonders bevorzugter Ausführung ist der Behälter durch eine Bandkassette gebildet, wobei das Testband aus einer Vorratskammer über eine Applikationsstelle in einen Abfall transportierbar ist und die Öffnung sich im Bereich der Vorratskammer befindet.

Eine weitere Gebrauchsverbesserung ergibt sich dadurch, dass das Testband an seiner von den Testfeldern abgewandten glatten Rückseite über die Dichtung gleitet, während die mit erhabenen Testfeldern versehene Vorderseite gegen die flexible Verschlussfolie randseitig anläuft.

Für den Einsatz einer Mehrzahl von disposiblen Teststreifen ergeben sich besondere Montagevorteile, wenn die Teststreifen vorzugsweise als Streifenstapel durch die Öffnung hindurch in den Behälter eingebracht sind.

Eine weitere Verbesserung lässt sich dadurch erzielen, dass der Behälter durch ein Streifenmagazin gebildet ist, wobei die Teststreifen aus einer Vorratskammer einzeln ausschiebbar sind. Hierbei ist es günstig, wenn ein jeweiliger Teststreifen beim Ausgeben an seiner von dem Testfeld abgewandten Rückseite über die Dichtung gleitet und vorderseitig gegen die flexible Verschlussfolie anläuft.

Generell kann die Öffnung vor dem Aufbringen der Verschlussfolie einen Einlass für besondere Einsatzteile des Behälters bilden, speziell für Trockenmittel, welche vorzugsweise in Form von funktionellen Verbundspritzgussteilen durch die Öffnung hindurch in den Behälter eingebracht sind. Wie bereits erwähnt, lassen sich auch ein Bandwickel oder Streifenstapel als vorgefertigte Einsatzteile vor dem Versiegeln der Öffnung problemlos in den Behälter einsetzen.

Um auch kleinere Leckagen wirkungsvoll zu vermeiden, ist es vorteilhaft, wenn die Verschlussfolie durch ein Anpresselement zur Minimierung des Durchlassspalts in Richtung der Dichtung unter Flächenpressung gehalten ist. Vorteilhafterweise umfasst das Anpresselement eine Feder, insbesondere eine Blattfeder, die bevorzugt aus Edelstahl besteht.

Eine weitere Vereinfachung ergibt sich dadurch, dass der Durchlassspalt beim Aufbringen der Verschlussfolie durch einen Abschnitt des Testbands oder eines Teststreifens oder durch ein entsprechend dimensioniertes Einlegeteil freigehalten bleibt, während im übrigen Bereich die Verschlussfolie entlang einer Siegellinie stoffdicht mit dem Behälter und/oder der Dichtung verbunden wird. Bevorzugt wird die Verschlussfolie auch auf das im Spalt befindliche Testband bzw. das Einlegeteil aufgesiegelt, so dass die Öffnung bis zum ersten Gebrauch umlaufend stoffdicht versiegelt bleibt. Dies lässt sich auf besonders einfache Weise dadurch erreichen, dass die Verschlussfolie durch ein mit einer Heißsiegelbeschichtung versehenes Trägermaterial vorzugsweise aus Aluminium gebildet ist. Weiterhin ist es günstig, wenn die Verschlussfolie auf eine die Öffnung begrenzende, vorzugsweise ebene Ringfläche an dem Behälter aufgesiegelt bzw. stoffschlüssig aufgebracht ist, wobei zumindest ein Segment der Ringfläche durch die Dichtung gebildet ist, und wobei die Verschlussfolie die Ringfläche vollflächig überspannt.

Herstellungstechnisch ist es von Vorteil, wenn die Dichtung als Einkomponenten-Spritzgussteil oder in Verbindung mit dem Behälter als Zweikomponenten-Spritzgussteil vorzugsweise aus einem thermoplastischen Elastomer (TPE) ausgebildet ist.

Um die Lagerstabilität zusätzlich zu verbessern, ist es vorteilhaft, wenn der mit dem Testband oder den Teststreifen bestückte Behälter in einer bei Gebrauch zu öffnenden stoffdichten Umverpackung gelagert ist.

Im Folgenden wird die Erfindung anhand der in der Zeichnung schematisch dargestellten Ausführungsbeispiele näher erläutert. Es zeigen
- Fig. 1: eine Testbandkassette für Blutzuckertests mit einer Folienabdichtung in einer aufgebrochenen Seitenansicht;
- Fig. 2: einen Ausschnitt der Testbandkassette nach Fig. 1 in perspektivischer Ansicht;
- Fig. 3: einen Kassettenbehälter der Testbandkassette mit umlaufender Dichtung in perspektivischer Ansicht;
- Fig. 4: einen Kassettenbehälter der Testbandkassette mit einem Dichtungssegment in perspektivischer Ansicht;
- Fig. 5: den mit Siegelfolie verschlossenen Behälter nach Fig. 4 und 5;
- Fig. 6: den Behälter nach Fig. 5 zusätzlich mit einem anpressenden Schließelement; und
- Fig. 7: ein Streifenmagazin für Blutzuckertests mit einer Folienabdichtung in einer aufgebrochenen, ausschnittsweisen Perspektive.

Die in der Zeichnung dargestellten diagnostischen Testeinheiten erlauben eine Vielzahl von Blutzuckerbestimmungen vor Ort an vom Patienten selbst entnommenen Blutproben. Um die als Testband 10 oder Teststreifen 12 bevorrateten Testträger vor Umwelteinflüssen abzuschirmen, ist ein Behältnis 14. vorgesehen, das eine mittels Dichtung 16 randseitig begrenzte Öffnung 18 aufweist, welche unter Freihaltung eines Durchlassspalts 20 zum Durchführen des Testträgers durch eine Verschlussfolie 22 versiegelt ist.

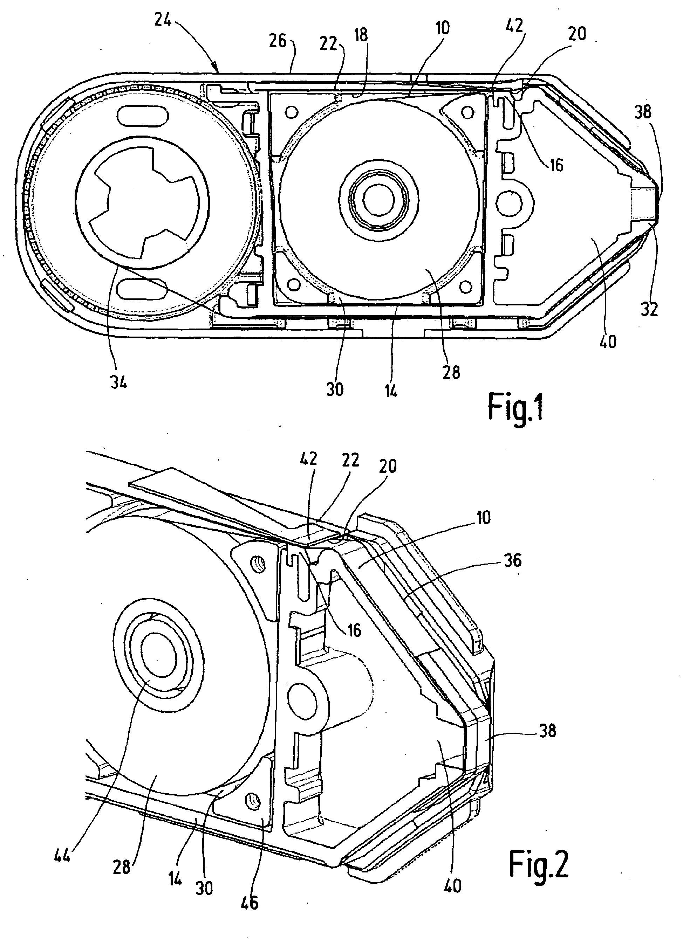

Fig. 1 zeigt eine Bandkassette 24 bestehend aus einem Kassettenkörper 26 und dem darin aufgenommenen Testband 10. Das Testband 10 ist in Form eines Vorratswickels 28 in der durch das Behältnis 14 begrenzten Aufnahmekammer 30 gelagert und lässt sich unter Umlenkung über eine von außen zugängliche Applikationsspitze 32 auf eine Abfallspule 34 vorspulen.

Wie auch aus Fig. 2 ersichtlich, weist das Testband 10 eine dünne Trägerfolie 36 auf, entlang welcher im Abstand voneinander eine Vielzahl von Testfeldern 38 aufgebaut sind. Die Testfelder 38 können somit nach Bedarf durch Bandvorschub im Bereich der Applikationsspitze 32 positioniert und dort gezielt mit Körperflüssigkeit (Blut, ggf. auch Gewebeflüssigkeit) beaufschlagt werden.

Die Testfelder 38 sind als vorgefertigte, etikettenartige Zuschnittteile auf das Trägerband 14 aufgeklebt und demzufolge gegenüber diesem erhöht. Sie können Trockenreagenzien enthalten, die auf den Analyten (Glucose) unter Farbänderung ansprechen und so einen optischen Nachweis erlauben. Zu diesem Zweck kann eine geräteseitige Messeinheit (nicht gezeigt) in den Kopfbereich 40 der Kassette 24 eingreifen.

Um die feuchtigkeitsempfindliche Testchemie auch über einen längeren Lagerzeitraum zu schützen, ist die Öffnung 18 der Vorratskammer 30 mit Ausnahme des über der Dichtung 16 liegenden Durchlassspalts 20 durch die Verschlussfolie 22 stoffdicht abgedichtet. Im Lagerzustand liegt ein testfeldfreier Trägerabschnitt des Trägerbands 36 im Spaltbereich, mit welchem die Verschlussfolie 22 vor dem ersten Gebrauch lösbar verbunden sein kann, so dass Leckagen minimiert werden. Um die Dichtwirkung weiter zu verbessern, ist ein Anpresselement 42 vorgesehen, welches eine Flächenpressung auf die Verschlussfolie 22 in Richtung der Dichtung 16 ausübt. Auf diese Weise wird das dazwischenliegende Trägerband 36 in die weiche Dichtung 16 gedrückt und von dieser rückseitig umschlossen und abgedichtet.

Beim Bandvorschub gleitet die glatte Rückseite des Trägerbands 36 über die Dichtung 16, während ein durchlaufendes erhöhtes Testfeld 38 gegen den Rand der flexiblen Verschlussfolie 20 anläuft und diese entgegen der Rückstellkraft des Anpresselements 42 abhebt. Um eine Abdichtung bei definierter Auszugskraft zu erreichen, sollten die Shorehärte der Dichtung 16 und deren konstruktive Gestaltung, die Reibwerte der eingesetzten Materialien und die Flächenpressung des Anpresselements 42 genau aufeinander abgestimmt sein.

Die solchermaßen abgedichtete Vorratskammer 30 kann zur weiteren Nutzungsoptimierung durch Einsetzen von Trockenmitteln konditioniert werden. Hierbei können Trockenmittel-Verbundspritzgussteile eine Zusatzfunktion beispielsweise als Hilfsteil beim Einsetzen und Positionieren des Bandwickels 28 erfüllen. Speziell können Lagerzapfen 44 oder Stützteile 46 ausgeformt werden. Zugleich kann durch solche Compoundmaterialien eine hohe Feuchteaufnahmekapazität und Aufnahmegeschwindigkeit erreicht werden. Durch eine geeignete Umverpackung der gesamten Kassette 24 durch ein dichtes Folienmaterial lässt sich die Lagerstabilität vor Gebrauch weiter steigern.

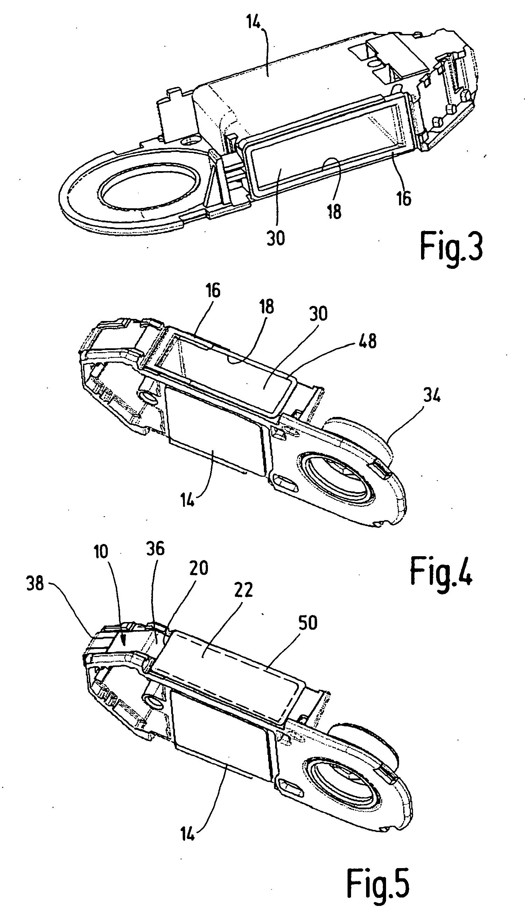

Wie am besten aus Fig. 3 und 4 ersichtlich, ist die durch die Siegelfolie 22 verschließbare Öffnung 18 so dimensioniert, dass im Zuge der Herstellung das Testband 10 in Form des Vorratswickels 28 inklusive eventueller Trockenmittel-Montagefunktionsteile komplett in die Vorratskammer 30 einsetzbar ist. Zweckmäßig ist hier eine Rechtecköffnung 18 vorgesehen, deren Öffnungslänge größer als der Ausgangsdurchmesser des Vorratswickels und deren Breite größer als die Testbandbreite ist.

In der Ausführung nach Fig. 3 ist eine umlaufende Rechteckprofildichtung 18 vorzugsweise aus einem TPE in eine entsprechende Randausnehmung eingesetzt. Dabei wird eine ebene Dichtfläche geschaffen, die das stoffdichte Aufsiegeln der Verschlussfolie 22 außerhalb des Durchlassspalts 20 vereinfacht.

Fig. 4 zeigt eine Abwandlung, bei der ein Dichtungssegment 16 dem Banddurchlass zugeordnet ist, während die übrige Randbegrenzung 46 der Öffnung 18 durch Wandungen des Behälters 14 gebildet wird. Die Herstellung einer solchen Struktur kann in einem einheitlichen Verfahren unter Einsatz einer Zweikomponenten-Spritzgusstechnik erfolgen. Hierbei ist durch geeignete Materialauswahl dafür zu sorgen, dass eine feste dichte Verbindung beim Spritzvorgang entsteht.

Der so gestaltete ebene Ringbereich 48 wird gemäß Fig. 5 mit einer Verschlussfolie 22 entlang eines etwa U-förmigen Siegelstreifens 50 heiß versiegelt. Die Versiegelung erfolgt entweder nur auf dem Dichtungsring 16 (Fig. 3) oder auf dem Dichtungssegment 16 und gleichzeitig auf dem Behältermaterial (Fig. 4). Die dünne flexible Verschlussfolie 22 kann durch eine Aluminiumfolie gebildet sein, die innenseitig mit einer schmelzbaren Heißsiegelbeschichtung versehen ist. Die Schmelztemperaturen aller am Siegelprozess beteiligten Materialien sind auf einem einheitlichen Temperaturniveau zu gewährleisten. Um den Durchlassspalt 20 freizuhalten, kann während des Heißsiegelvorgangs entweder das Trägerband 36 oder ein dessen Geometrie entsprechendes Einlegeteil mit eingesiegelt werden. Beim Heißsiegeln entsteht dann auf der ganzen Fläche des Siegelstreifens 48 mit Ausnahme des eingelegten Bandes oder Einlegeteils eine dichte Heißsiegelverbindung zwischen Behältnis 14 bzw. Dichtung 16 und der heißsiegelfähigen Innenseite der Aluminiumfolie 22. Nach dem Heißsiegelvorgang kann das ggf. ebenfalls klebend verbundene Trägerband 36 von leichten Anhaftungen durch geringfügigen Weitertransport zweckmäßig beim Erstgebrauch befreit werden. Falls vorhanden, wird das Einlegeteil ausgezogen und so der Durchlassspalt 20 freigelegt.

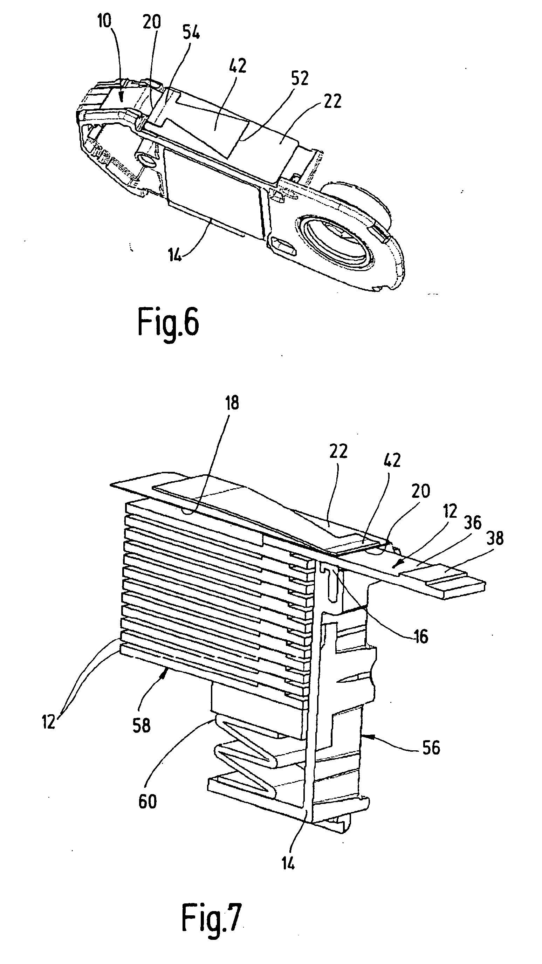

Fig. 6 veranschaulicht zusätzlich die Funktion des Anpresselements 42. Dieses kann durch eine Blattfeder gebildet sein, die an einem Ende 52 kassettenseitig abgestützt ist und am breitflächigen freien Ende 54 unter Vorspannung auf die Siegelfolie über dem Durchlassspalt 20 aufdrückt. Um Materialermüdungen auch bei wechselnder Klimabelastung zu minimieren, kann eine Edelstahlfeder verwendet werden.

Eine für den Einsatz von Teststreifen 12 vorgesehene Erfindungsvariante ist in Fig. 7 dargestellt. Die konstruktive Gestaltung der Durchzugdichtung 16, 22 entspricht im Wesentlichen der vorstehend erläuterten Ausführung, so dass für gleiche Teile gleiche Bezugszeichen verwendet sind. Anstelle einer Bandkassette ist hier ein nur ausschnittsweise gezeigtes Streifenmagazin 56 vorgesehen, welches ein rechteckförmiges Vorratsbehältnis 14 zur Aufnahme eines Stapels 58 von Teststreifen 12 umfasst. Jeder Teststreifen 12 besitzt auf einem Abschnitt einer Trägerfolie 36 ein Testfeld 38 zum Aufbringen der Körperflüssigkeit. Der Streifenstapel 58 wird vor dem Aufsiegeln der Verschlussfolie 22 als komplettes Paket durch die Öffnung 18 hindurch in den Behälter 14 eingesetzt und dann eingesiegelt, wobei durch ein Einlegeteil der Durchlassspalt 20 freigehalten wird. Am Behälterboden ist eine Federmimik 60 abgestützt, um den Stapel 58 zu dem oberen Entnahmebereich hin nachzustellen. Die Entnahme jeweils des oberen Streifens 12 kann durch eine nicht gezeigte Schiebemechanik erfolgen, wodurch der betreffende Streifen wie gezeigt in Streifenlängsrichtung durch den Durchlassspalt 20 hindurch ausgeschoben und zum Gebrauch bereitgestellt wird. Dabei wird das Anpresselement 42 nur zur Streifenausgabe angehoben, während über die sonstige Zeit kein Streifen 12 in dem Durchlassspalt 20 liegt und somit eine hohe Dichtigkeit gewährleistet ist.

Sowohl die Bandkassette 24 als auch das Streifenmagazin 56 lassen sich als Verbrauchsmaterial in ein Aufnahmefach eines Handgeräts einwechseln, um den Testvorgang ggf. einschließlich Probennahme automatisieren zu können.

## Patentansprüche

1. Diagnostische Testeinheit zur Untersuchung einer Körperflüssigkeit, insbesondere für Blutzuckertests, mit einem mit Testfeldern (38) zur Applikation der Körperflüssigkeit versehenen Testband (10) und einem das Testband (10) enthaltenden Behälter (14), wobei eine Öffnung (18) des Behälters (14) zum Ausgeben von Testband (10) zumindest bereichsweise durch eine Dichtung (16) begrenzt ist, **dadurch gekennzeichnet, dass** die Öffnung (18) durch eine Verschlussfolie (22) gegenüber der Umgebung des Behälters (14) abgeschirmt ist, wobei die Verschlussfolie (22) auf eine die Öffnung (18) begrenzende Ringfläche (48) an dem Behälter (14) aufgesiegelt ist und zumindest ein Segment der Ringfläche (48) durch die Dichtung (16) gebildet ist, und wobei das Testband (10) durch einen Durchlassspalt (20) zwischen der Verschlussfolie (22) und der Dichtung (16) hindurchgeführt ist.

2. Testeinheit nach Anspruch 1, **dadurch gekennzeichnet, dass** das Testband (10) vorzugsweise in Form eines Bandwickels (28) durch die Öffnung (18) hindurch in den Behälter (14) eingesetzt ist.

3. Testeinheit nach Anspruch 1 oder 2, **dadurch gekennzeichnet, dass** das Testband (10) zwischen aufeinander folgenden Testfeldern (38) freie Trägerabschnitte (36) aufweist, und dass im Lagerzustand ein testfeldfreier Trägerabschnitt (36) in dem Durchlassspalt (20) liegt.

4. Testeinheit nach einem der Ansprüche 1 bis 3, **dadurch gekennzeichnet, dass** der Behälter (14) durch eine Bandkassette (24) gebildet ist, wobei das Testband (10) aus einer Vorratskammer (30) über eine Applikationsstelle (32) in einen Abfall (34) transportierbar ist und die Öffnung (18) im Bereich der Vorratskammer (30) angeordnet ist.

5. Testeinheit nach einem der Ansprüche 1 bis 4, **dadurch gekennzeichnet, dass** das Testband (10) an seiner die erhabenen Testfelder (38) tragenden Vorderseite gegen die flexible Verschlussfolie (22) randseitig anläuft.

6. Diagnostische Testeinheit zur Untersuchung einer Körperflüssigkeit, insbesondere für Blutzuckertests, mit einer Mehrzahl von Teststreifen (12), die jeweils mindestens ein Testfeld (38) zur Applikation der Körperflüssigkeit aufweisen und einem die Teststreifen (12) aufnehmenden Behälter (14), wobei eine Öffnung (18) des Behälters (14) zum einzelnen Ausgeben von Teststreifen (12) zumindest bereichsweise durch eine Dichtung (16) begrenzt ist, **dadurch gekennzeichnet, dass** die Öffnung (18) durch eine Verschlussfolie (22) gegenüber der Umgebung des Behälters (14) abgeschirmt ist, wobei die Verschlussfolie (22) auf eine die Öffnung (18) begrenzende Ringfläche (48) an dem Behälter (14) aufgesiegelt ist und zumindest ein Segment der Ringfläche (48) durch die Dichtung (16) gebildet ist, und wobei die Teststreifen (12) zum Ausgeben durch einen Durchlassspalt (20) zwischen der Verschlussfolie (22) und der Dichtung (16) hindurchführbar sind.

7. Testeinheit nach Anspruch 6, **dadurch gekennzeichnet, dass** die Teststreifen (12) vorzugsweise als Streifenstapel (58) durch die Öffnung (18) hindurch in den Behälter (14) eingesetzt sind.

8. Testeinheit nach einem der Ansprüche 1 bis 7, **dadurch gekennzeichnet, dass** die Öffnung (18) vor dem Aufbringen der Verschlussfolie (22) einen Einlass für Einsatzteile des Behälters (14) bildet.

9. Testeinheit nach einem der Ansprüche 1 bis 8, **dadurch gekennzeichnet, dass** Trockenmittel vorzugsweise in Form von funktionellen Verbundspritzgussteilen (44,46) durch die Öffnung (18) hindurch in den Behälter (14) eingebracht sind.

10. Testeinheit nach einem der Ansprüche 1 bis 9, **dadurch gekennzeichnet, dass** die Verschlussfolie (22) durch ein Anpresselement (42) zur Minimierung des Durchlassspalts (20) in Richtung der Dichtung (16) unter Flächenpressung gehalten ist.

11. Testeinheit nach einem der Ansprüche 1 bis 10, **dadurch gekennzeichnet, dass** das Anpresselement (42) eine Feder, insbesondere eine Blattfeder umfasst.

12. Testeinheit nach einem der Ansprüche 1 bis 11, **dadurch gekennzeichnet, dass** der Durchlassspalt (20) beim Aufbringen der Verschlussfolie (22) durch einen Abschnitt des Testbands (10) oder eines Teststreifens (12) oder durch ein entsprechend dimensioniertes Einlegeteil freigehalten bleibt.

13. Testeinheit nach einem der Ansprüche 1 bis 12, **dadurch gekennzeichnet, dass** die Verschlussfolie (22) entlang einer Siegellinie (50) stoffdicht mit dem Behälter (14) und/oder der Dichtung (16) verbunden ist.

14. Testeinheit nach einem der Ansprüche 1 bis 13, **dadurch gekennzeichnet, dass** die Verschlussfolie (22) durch ein mit einer Heißsiegelbeschichtung versehenes Trägermaterial vorzugsweise aus Aluminium gebildet ist.

15. Testeinheit nach einem der Ansprüche 1 bis 14, **dadurch gekennzeichnet, dass** der mit dem Testband (10) oder den Teststreifen (12) bestückte Behälter (14) in einer bei Gebrauch zu öffnenden stoffdichten Umverpackung gelagert ist.

## Claims

1. Diagnostic test unit for analysing a body fluid in particular for blood sugar tests comprising a test tape (10) provided with test fields (38) for applying the body fluid and a container (14) containing the test tape (10), wherein an opening (18) of the container (14) for dispensing test tape (10) is at least in some areas bordered by a seal (16), **characterized in that** the opening (18) is screened by a closing foil (22) from the surroundings of the container (14), where the closing foil (22) is sealed onto a ring face (48) on the container (14) which borders the opening (18) and at least one segment of the ring face (48) is formed by the seal (16), and where the test tape (10) is passed through a passage gap (20) between the closing foil (22) and the seal (16).

2. Test unit according to claim 1, **characterized in that** the test tape (10) is inserted preferably in the form of a tape roll (28) through the opening (18) into the container (14).

3. Test unit according to claim 1 or 2, **characterized in that that** the test tape (10) has free carrier sections (36) between successive test fields (38) and that a test field-free carrier section (36) is in the passage gap (20) in a storage state.

4. Test unit according to one of the claims 1 to 3, **characterized in that** the container (14) is formed by a tape cassette (24) wherein the test tape (10) can be transported out of a storage chamber (30) over an application site (32) into a waste (34) and the opening (18) is located in the area of the storage chamber (30).

5. Test unit according to one of the claims 1 to 4, **characterized in that** the front side of the test tape (10) carrying the raised test fields (38) runs towards the border of the flexible closing foil (22).

6. Diagnostic test unit for analysing a body fluid in particular for blood sugar tests comprising a plurality of test strips (12) which each have at least one test field (38) for applying the body fluid and a container (14) for receiving the test strips (12) wherein an opening (18) of the container (14) for individually dispensing test strips (12) is bordered by a seal (16) at least in some areas, **characterized in that** the opening (18) is screened by a closing foil (22) from the surroundings of the container (14), where the closing foil (22) is sealed onto a ring face (48) on the container (14) which borders the opening (18) and at least one segment of the ring face (48) is formed by the seal (16), and where the test strips (12) can be passed through a passage gap (20) between the closing foil (22) and the seal (16) for dispensing.

7. Test unit according to claim 6, **characterized in that** the test strips (12) are introduced into the container (14) through the opening (18) preferably as a stack of strips (58).

8. Test unit according to one of the claims 1 to 7, **characterized in that** the opening (18) forms an inlet for insert parts of the container (14) before the closing foil (22) is applied.

9. Test unit according to one of the claims 1 to 8, **characterized in that** desiccants preferably in the form of functional composite injection-moulded parts (44, 46) are introduced through the opening (18) into the container (14).

10. Test unit according to one of the claims 1 to 9, **characterized in that** the closing foil (22) is held by a pressing element (42) under surface pressure to minimize the passage gap (20) towards the seal (16).

11. Test unit according to one of the claims 1 to 10, **characterized in that** the pressing element (42) comprises a spring and in particular a leaf spring.

12. Test unit according to one of the claims 1 to 11, **characterized in that** when the closing foil (22) is applied, the passage gap (20) is kept free by a section of the test tape (10) or of a test strip (12) or by an appropriately dimensioned insert.

13. Test unit according to one of the claims 1 to 12, **characterized in that** the closing foil (22) is joined along a sealing line (50) with the container (14) and/or the seal (16) in a material-tight manner.

14. Test unit according to one of the claims 1 to 13, **characterized in that** the closing foil (22) is formed by a carrier material preferably made of aluminium which is provided with a heat-seal coating.

15. Test unit according to one of the claims 1 to 14, **characterized in that** the container (14) which is loaded with the test tape (10) or the test strips (12) is stored in a tight outer packaging which is to be opened for use.

## Revendications

1. Unité de test de diagnostic pour analyser un liquide corporel, en particulier pour tests de glycémie, avec une bande de test (10) pourvue de zones de test (38) pour appliquer le liquide corporel, et avec un récipient (14) contenant la bande de test (10), une ouverture (18) du récipient (14) pour distribuer la bande de test (10) étant délimitée au moins partiellement par un joint (16), **caractérisée en ce que** l'ouverture (18) est protégée par rapport à l'environnement du récipient (14) par une feuille d'obturation (22), la feuille d'obturation (22) étant scellée sur le récipient (14) sur une surface annulaire (48) délimitant l'ouverture (18) et au moins un segment de la surface annulaire (48) étant formé par le joint (16), et la bande de test (10) étant guidée à travers une fente de passage (20) entre la feuille d'obturation (22) et le joint (16).

2. Unité de test selon la revendication 1, **caractérisée en ce que** la bande de test (10) est placée de préférence sous forme de bobine de bande (28) à travers l'ouverture (18) dans le récipient (14).

3. Unité de test selon la revendication 1 ou 2, **caractérisée en ce que** la bande de test (10) présente entre zones de test (38) successives des portions de support (36) libres et **en ce qu'**à l'état de stockage, une portion de support (36) exempte de zone de test est située dans la fente de passage (20).

4. Unité de test selon l'une des revendications 1 à 3, **caractérisée en ce que** le récipient (14) est formé par une cartouche de bande (24), la bande de test (10) pouvant être transportée depuis un compartiment de réserve (30), via un point d'application (32), dans une évacuation (34), l'ouverture (18) étant placée au niveau du compartiment de réserve (30).

5. Unité de test selon l'une des revendications 1 à 4, **caractérisée en ce que** la bande de test (10) bute au niveau de sa face supérieure portant les zones de test (38) en relief contre le bord de la feuille d'obturation (22) flexible.

6. Unité de test de diagnostic pour analyser un liquide corporel, en particulier pour tests de glycémie, avec une pluralité de bandelettes de test (12) présentant chacune au moins une zone de test (38) pour appliquer le liquide corporel, et avec un récipient (14) recevant les bandelettes de test (12), une ouverture (18) du récipient (14) pour distribuer individuellement les bandelettes de test (12) étant délimitée au moins partiellement par un joint (16), **caractérisée en ce que** l'ouverture (18) est protégée par rapport à l'environnement du récipient (14) par une feuille d'obturation (22), la feuille d'obturation (22) étant scellée sur le récipient (14) sur une surface annulaire (48) délimitant l'ouverture (18) et au moins un segment de la surface annulaire (48) étant formé par le joint (16), et les bandelettes de test (12), en vue de leur distribution, étant guidées à travers une fente de passage (20) entre la feuille d'obturation (22) et le joint (16).

7. Unité de test selon la revendication 6, **caractérisée en ce que** les bandelettes de test (12) sont placées de préférence sous forme de pile de bandelettes (58) à travers l'ouverture (18) dans le récipient (14).

8. Unité de test selon l'une des revendications 1 à 7, **caractérisée en ce que** l'ouverture (18) forme avant l'application de la feuille d'obturation (22) une entrée pour des pièces d'insertion du récipient (14).

9. Unité de test selon l'une des revendications 1 à 8, **caractérisée en ce que** des moyens de dessiccation sont placés de préférence sous forme de pièces fonctionnelles en composite moulé par injection (44, 46) à travers l'ouverture (18) dans le récipient (14).

10. Unité de test selon l'une des revendications 1 à 9, **caractérisée en ce que** la feuille d'obturation (22) est maintenue sous pression superficielle par un élément de pression (42) destiné à minimiser la fente de passage (20) en direction du joint (16).

11. Unité de test selon l'une des revendications 1 à 10, **caractérisée en ce que** l'élément de pression (42) comprend un ressort, en particulier un ressort à lames.

12. Unité de test selon l'une des revendications 1 à 11, **caractérisée en ce que** pendant l'application de la feuille d'obturation (22), la fente de passage (20) est maintenue libre par une portion de la bande de test (10) ou d'une bandelette de test (12), ou par une pièce d'insertion de dimensions correspondantes.

13. Unité de test selon l'une des revendications 1 à 12, **caractérisée en ce que** le long d'une ligne de scellage (50), la feuille d'obturation (22) est reliée hermétiquement au récipient (14) et/ou au joint (16).

14. Unité de test selon l'une des revendications 1 à 13, **caractérisée en ce que** la feuille d'obturation (22) est formée par un matériau support de préférence en aluminium, muni d'un revêtement scellé à chaud.

15. Unité de test selon l'une des revendications 1 à 14, **caractérisée en ce que** le récipient (14) équipé de la bande de test (10) ou des bandelettes de test (12), est conservé dans un suremballage hermétique, à ouvrir en cas d'utilisation.
